# EUROPEAN PATENT APPLICATION

(11) **EP 1 972 348 A1**
(43) Date of publication of application: **24.09.2008**
(21) Application number: 07290319.8
(22) Date of filing: 14.03.2007
(51) Int. Cl.: A61K 39/155, C07K 14/135

(54) **Novel vaccine composition for the treatment of respiratory infectious diseases**

(71) Applicant: PIERRE FABRE MEDICAMENT, 92100 Boulogne-Billancourt (FR)
(72) Inventor: Hurtado, Pepi, 08960 Sant Just Desvern - Barcelona (ES); Ferret, Eulalia, 08820 El Prat de Llobregat - Barcelona (ES); Perez, Amadeo, 08028 Barcelona - Barcelona (ES); Asin, Miguel Angel, 08290 Cerdanyola del Valles (ES); Libon, Christine, 31520 Ramonville Saint Agne (FR); Nguyen, Ngoc Thien, 31180 Rouffiac Tolosan (FR)
(74) Representative: Warcoin, Jacques

(57) **Abstract**

The present invention relates to a composition comprising a microencapsulated antigen having a native conformational epitope, particularly an antigen derived from the G protein of the human respiratory syncytial virus (RSV) encapsulated in poly(D,L-lactide-co-glycolide) copolymers (PLGA) microspheres. The present invention further relates to a method for producing such a composition and the use of these compositions for the preparation of a vaccine or immunogenic composition intended for the prevention or the treatment of RSV infection in mammals.

## Description

The present invention relates to a composition comprising a microencapsulated antigen having a native conformational epitope, particularly an antigen derived from the G protein of the human respiratory syncytial virus (RSV) encapsulated in poly(D,L-lactide-co-glycolide) copolymers (PLGA) microspheres. The present invention further relates to a method for producing such a composition and the use of these compositions for the preparation of a vaccine or immunogenic composition intended for the prevention or the treatment of RSV infection in mammals.

RSV, a *Pneumovirus* of the family *Paramyxoviridae,* is a major respiratory pathogen in the two extreme age of human life. RSV is the main cause of severe bronchiolitis and pneumonia in newborns and in young infants. It is increasingly recognized that elderly and immunocompromised patients are also concerned about RSV infection. Morbidity, mortality and hospitalization rates in these populations are comparable to Influenza infection. RSV does not induce a long life immunity and reinfections are common throughout life.

Today only three drugs have been approved for use for prophylactic treatment of RSV infection. Ribavirin, a nucleoside analogue, provides an aerosol treatment for serious RSV infection in hospitalized children. The aerosol route of administration, the toxicity (risk of teratogenicity), the cost and the highly variable efficacy limit its use. The other two, RespiGam and SYNAGIS, respectively polyclonal and monoclonal antibody, are mainly used in high-risk new borns and infant.

Attempts to develop a safe and effective RSV vaccine have all met with failure thus far. Inactivated vaccines not only failed to protect against disease, but in contrast disease (immunopathology) was exacerbated and potentiated during subsequent RSV infection. Live attenuated vaccines have been tried with limited success.

Clearly there is a need for an efficacious non-toxic and easy to administer drug against RSV replication. For this purpose, it is important to formulate a vaccine composition showing a great efficacy, i.e. with a good anti-viral protection (neutralizing antibodies) as well as a persistence of protective activity during the time, with limited injections, preferably by only a single injection.

The RSV surface glycoproteins, attachment G and fusion F proteins are recognized as the most important antigens that conferred protection against RSV challenge. A vaccine based on a conserved central domain of RSV G protein namely G2Na was developed by the applicant. In this application, particular methods of encapsulation of an antigen in a synthetic microsphere will be described. Surprisingly, a single injection of this formulation is capable of inducing strong RSV primary antibody response, followed later on by a boost of secondary response which maintained for more than 120 days.

According to a first aspect, the invention concerns a composition comprising at least an antigen microencapsulated in microspheres for a controlled release of the antigen, said at least one antigen presenting a native conformational epitope responsible of the biological activity of said antigen, said native conformational epitope being conserved in the microspheres.

By the expression "microspheres", it must be understood spherical particles sized from 1µm to few hundred µm. The expression "microparticles" includes both "microspheres" and "microcapsules". In the present specification, the expression "microspheres" is used but it must be understood that, when the substitution with a "microcapsule" is possible and of interest according to the man skilled in the art, the use of "microspheres" and "microcapsules" is equivalent.

By the expression "encapsulated" or "microencapsulated", it must be understood a drug or drugs enclosed or embedded in particles for protection or for modified release.

In the process of microencapsulation of an antigen within microspheres, such as for example microspheres made by poly(D,L-lactide-co-glycolide) copolymers (PLGA), it was not predictable that native conformation of the antigen would be conserved in its active form and in particular disulfide bridges would not be altered.

Moreover, it is known in literature that complex proteins are unstable when formulated into microparticles such as PLGA microspheres. De Weert et al (Pharmaceutical Research, 2000, 17:1159-1167) and Jacob et al (Indian Journal Pharmaceutical Science, 2006, 68:154-163) have reviewed numerous problems of microencapsulating proteins in PLGA microspheres: protein degradation during preparation, storage and release from PLGA microspheres.

A first particularity of the invention is to allow the preservation of the conformational epitope as it was not evident that a conformational epitope would be conserved after its microencapsulation in microspheres.

Generally, linear epitope consists of about 6 to 10 adjacent amino acids on a protein molecule that is recognized by an antibody. In contrast, conformational epitope consists of amino acids that are not arranged sequentially, particularly due to the presence of one or more disulfide bridges in the epitote. Here the antibody recognizes only the 3-dimensional structure. When a protein molecule folds into a three dimensional structure the amino acids forming the epitope are juxtaposed enabling the antibody directed against this conformational epitope to recognize the sequence.

The differences between linear or conformational epitope is of significance in immunological or in vaccine applications. To obtain efficient immunization against microorganism having conformational epitope, it is consequently necessary to maintain this conformation in the antigen used after its administration and its release in the host organism.

The present invention is directed to a composition comprising at least one antigen microencapsulated in a single population of microspheres, characterized in that said at least one antigen is a respiratory syncytial virus (RSV) antigen comprising a native conformational epitope.

Preferably, this nativeconformational epitope is associated to the formation of at least one disulfide bridge between two cysteine residues situated in the amino acid sequence of this epitope.

In a preferred aspect, the composition according to the invention comprises a conformational epitope which is a fragment of at least 10 amino acids length of the G protein of the human RSV type A or B.

In a more preferred aspect, said at least one antigen comprises the fragment aa174-187 of the G protein of the human RSV type A or B.

G2Na is a 11.20 kDa protein derived from the central conserved domain of RSV A G attachment protein corresponding to aa130-230. A vaccine based on G2Na was developed by the applicant and is described, for example, in the patent applications WO95/27787 or WO96/14416.

Several protective epitopes were identified in the amino acid sequence aa130-230 of the G protein of the human RSV type A (sequence called "G2Na" or "G2A") (Plotnicky *et al*, J. Virol., 1999, 73:5637). Among these protective epitope, the fragment aa174-187 (called "G1A") has been demonstrated as being one of the best protective epitope (see the PCT patent document WO 95/27787, example 7).

More particularly, the peptide aa130-230 of the human RSV A or B G protein, or fragment thereof containing at least the sequence aa174-187, is a highly conformational protein as it contains the highly conserved "cysteine noose" domain formed by the 2 cysteine residues, respectively residues Cys₁₇₆ and Cys₁₈₂ (Simard et al., Vaccine, 1997, vol.15, No.4, pp.4e23-432)

The numbering of the Cys residues in the following specification is done in regards with the whole sequence of the RSV A G protein (SEQ ID No. 5) . For more clarity, the following table 1 illustrates the corresponding numbering with the peptide G2Na sequence (SEQ ID No.1).

**Table 1**

| | | | | |
|---|---|---|---|---|
| RSV A G protein (SEQ ID No.5) | | Cys176 | Cys182 | |
| G2Na peptide (SEQ ID No.1) | | Cys 47 | Cys 53 | |

The native conformation has a disulfide bridge defined by residues Cys₁₇₆ - Cys₁₈₂.

This conformational epitope is critical for the biological activities of the antigen containing said epitope, such as the protective activity of this antigen. A specific monoclonal antibody, namely 18D1 (the generation of said antibody is disclosed in the following examples), which recognizing the native cysteine noose has been generated by the applicant. This monoclonal antibody neutralizes *in vitro* and *in vivo* RSV.

If this disulfide bridge is lacking or "miss-pairing" (see the presence of the Cys₁₇₃ and Cys₁₈₆ which can form another disulfide bridge), not only the peptides are not recognized by 18D1 but if animals are immunized with these miss-folding peptides, protective efficacy of the induced antibodies is dramatically reduced compared to the those immunized with the native formed peptides.

Other methods are well known by the skilled person to control whether the conformational epitope is preserved or not after its preparation (such as encapsulation into microspheres) and, if so, its release in the host organism.

For example, the Circular dichroism (CD) spectroscopy whichmeasures differences in the absorption of left-handed polarized light versus right-handed polarized light which arise due to structural asymmetry can be used. The absence of regular structure results in zero CD intensity, while an ordered structure results in a spectrum which can contain both positive and negative signals. Circular dichroism spectroscopy is particularly good for demonstrating comparability of solution conformation after changes in manufacturing processes or formulation or for studying the conformational stability of a protein under stress (thermal stability, pH stability, and stability to denaturants and how this stability is altered by buffer composition or addition of stabilizers and excipients).

It was very surprising that the majority of microencapsulated G2Na antigens, including those close to the surface of the microspheres had their conformational epitopes intact especially the ones with at least a disulfide bridge. It was confirmed *in vivo* that microspheres-G2Na induced protective antibodies response that neutralized the RSV in animal.

In a more preferred embodiment, the present invention relates to a composition according to the invention, wherein said native conformational epitope comprises at least the disulfide bridge between the residue Cys in position 176 and the residue Cys in position 182 of the G protein of the human RSV type A or B.

Other interesting fragments could be mentioned such as the peptide resulting from the covalent coupling between the fragment aa140-190 and aa144-158, between the fragment aa140-190 and aa144-159 or between the fragment aa140-192 and aa144-158 of the G protein of the human RSV type A or B (called G20a, G20ap and G23a in the PCT patent application WO 03/010317). These two fragments are of particular interest in the preparation of a composition according to the invention for treating newborns and young infants. Other peptides could be mentioned such as the ones described in the published application WO 96/14416 or any other fragments known by the man skilled in the art. According to another aspect, such fragments could be glycosylated or not as described in the patent application WO 02/058725.

It is preferred that said at least one antigen comprises or is the antigen selected from the group consisting of:
a) the sequence aa174-187, aa130-230, aa171-187, aa140-190, aa158-190, aa140-192, aa140-194, aa140-196, aa140-198, aa140-200, of the G protein of the human RSV type A or B;
b) the peptide resulting from the covalent coupling between the fragment aa140-190 and aa144-158, or resulting from the covalent coupling between the fragment aa140-190 and aa144-159, or resulting from the covalent coupling between the fragment aa140-192 and aa144-158 of the G protein of the human RSV type A or B; and
c) the sequences as defined in a) or b) wherein the cysteine residue in position 173 and/or 186 has been replaced by a serine residue.

In a more preferred embodiment, said at least one antigen comprises or consists in the peptide G2Na having the sequence SEQ ID No. 1, or any fragment thereof comprising at least the fragment aa174-187 (corresponding to the fragment.aa45-58 of the sequence SEQ ID No.1).

In another preferred embodiment, the invention relates to a composition of the invention wherein said at least one antigen is covalently coupled to the triamino-acids peptide having the sequence MEF.

Preferably, said at least one antigen comprises or consists in the peptide MEFG2Na having the sequence SEQ ID No. 2.

MEFG2Na, a 11.55 kDa protein, corresponds to the antigen G2Na associated with the triamino-acid peptide MEF as described in the patent application WO 2006/018527 published on 23 February 2006.. This protein is produced recombinantly with high yield in *E. coli.* MEFG2Na adsorbed in Alum showed to be immunogenic but this formulation needs to be injected several times in order to boost and maintain antibody responses level in mammals for several months.

Another particularity of the invention is that, in the composition according to the present invention, the antigen is dispersed in the microspheres in the absence of any adjuvant.

According to another embodiment, the composition according to the invention also comprises at least a second antigen. Said second antigen could be also microencapsulated or not.

In a first embodiment, the composition according to the invention comprises a second antigen, said second antigen being microencapsulated in different microspoheres than the ones used for the first antigen.

Different microspheres could be, as example, formulated by a general double emulsion and solvent evaporation-extraction technique; procedure conditions and type of polymer may be adjusted, if needed.

In a second embodiment, the composition according to the invention comprises a second antigen, said second antigen being free or adsorbed on aluminum salts and/or aluminum phosphate and/or blank PLGA microspheres and/or antigen loaded PLGA microspheres in the said composition.

The choice to microencapsulate or not the second antigen is dictated by the nature of the antigen. The man skilled in the art will be able to decide if a microencapsulation or encapsulation procedure is necessary or not.

In the case of adsorption of the second antigen, it can be adsorbed on aluminum salts such as aluminum hydroxide (preferred) or aluminum phosphate. It can also be adsorb on microspheres surface by incubation of both PLGA microspheres and antigen molecules in an adequate medium. Such PLGA microspheres could be blank PLGA microspheres or antigen loaded PLGA microspheres.

More particularly, in consideration to the disease the invention intends to treat, it could also be envisaged to use a second antigen directly linked with the RSV. Several RSV antigens, such as Matrix (M) protein, Small hydrophobic (SH) glycoprotein, Small envelope (M2), Phosphoprotein (P), Large polymerase complex (L) and G glycoprotein can be used and, the F protein is the preferred one in terms of mediating protective antibody responses against RSV.

The F protein remains highly conserved across both major strains of RSV (subgroup A and subgroup B). The F protein is synthesized as a non-fusogenic 67 kDa precursor (F0) that undergoes proteolytic cleavage by furin to produce two disulfide-linked polypeptides, F1 and F2. The F protein enters the cell membrane via the N-terminus of the F 1 polypeptide, whereas the transmembrane segment is located close to the C-terminus. Adjacent to these two regions are two heptad repeat sequences, denoted HR-C and HR-N, that form a stable trimer of hairpin-like structures that undergo a conformational change to enable the viral and cell membranes to be apposed before viral entry.

According to the invention, the second antigen consists in the protein F, or an immunogenic active fragment thereof having at least 10 amino acid residues, of RSV A (SEQ ID No.3), of RSV B (SEQ ID No.4), or of RSV A and RSV B.

Biodegradable polymers such as polylactide-co-glycolides (PLGA) have been used to microencapsulate antigens for parenteral delivery in order to achieve a stable and therapeutically adequate level of antigen over an extended period of time, thus in order to achieve a long-lasting immune response. On the other hand, the use of microencapsulation to protect sensitive antigens and other type of molecules against degradation is well known in the art.

A number of techniques to produce microparticles have been described in the prior art. Due to the hydrophilic nature of the most antigen drugs, water in oil in water (w/o/w) double emulsion and solvent extraction-evaporation technique is frequently used for encapsulating antigens.

The drug release profile for a microparticle is dependent on numerous factors, including physicochemical properties of the polymers used, interactions among polymer-drug-excipients and/or morphology and composition of the resulting microparticles. The antigen release profile from microspheres will be then related to the immune response induced in animals or humans.

Traditional immunization schedules require a primary and one or more booster immunizations to achieve protective immunity. That means an antigen provided to the immune system in discrete pulses. Previous investigators have attempted to convert multiple dose immunization schedules to single dose schedules using controlled release antigen delivery systems comprising biodegradable microparticles. To mimic multiple dose immunization schedules, some researchers have proposed administration of alum adjuvant followed by controlled release formulations (e.g. PLGA microspheres). Other inventors have claimed that mixing populations of microparticles which release their bursts of antigens at multiple intervals will mimic multiple dose immunization schedules. Such populations of microparticles may be composed of microparticles made of different polymers with different degradation rates.

The present invention solves many of the problems associated with current immunization methods. Contrary to the teachings in the prior art, the present invention provides a biphasic immune response against the antigen released from microspheres, which are prepared using the method described here. According to the present invention, it has been surprisingly proved that such induction of immune responses is comparable to either that induced by subsequent administration of alum adjuvant and antigen-loaded microparticles or administration of a combination of microparticles made of different polymers.

More particularly, the composition according to the invention is characterized in that the controlled release is a biphasic release, thus mimicking a multiple dose schedule vaccination.

More particularly, according a preferred embodiment of the invention, the microspheres, respectively for each antigen, are selected in a single population of particles in terms of polymeric composition.

One aspect of the invention is the composition of the present invention comprises poly(D,L-lactide-co-glycolide) (PLGA) microspheres microencapsulating antigen, G2Na and/or MEFG2Na, wherein the ratio of lactide to glycolide is from about 50:50 to 80:20 molar ratio (lactide:glycolide); the inherent viscosity of PLGA is about 0.15 to 0.70 dl/g; the average diameter of the microspheres is in the range of 5 to 35 µm; the antigen experimental loading is from about 0.5 to 5 % (w/w), the microencapsulation efficiency being greater than 65 %; the microspheres show a porous surface; the antigen's integrity is conserved.

More particularly, the microspheres are incorporating from about 0.5 to 5 %, preferably from about 0.5 to 2 % of antigen. Preferably, said antigen contains at least the fragment aa174-187 of the human VRS type A or B G protein, such as G2Na or MEFG2Na antigen.

The present invention also relates to a composition according to the present invention, or a composition of the invention wherein the microencapsulated antigens are obtained by the hereinafter method, further comprising a pharmaceutically acceptable carrier or excipient, as a medicament.

The present invention also relates to a composition according to the present invention, or obtained by the hereinafter method, as a vaccine or an immunogenic composition.

Accordingly, it is also an object of the present invention to provide a method for producing a microencapsulated antigen or vaccine formulation which does not include any adjuvant and which consists of a single population of particles in terms of polymeric composition.

Thus, another aspect of the invention is a method for microencapsulating antigen in PLGA microspheres, comprising: (a) dissolving antigen, including or not including an antigen stabilizer, in a phosphate buffer solution of about neutral pH; (b) dissolving PLGA copolymer in an organic solvent; (c) adding (a) to (b) to produce a first emulsion; (d) adding the emulsion of step (c) to an aqueous solution containing a viscosizer agent, a surfactant and an osmotic agent to produce a second emulsion; (e) hardening and harvesting the resulting microspheres of step (f) and drying the microspheres comprising microencapsulated antigen.

More particularly, the invention claims a method for producing a composition as above described, characterized in that it is comprising the following steps:
a) dissolving the antigen in a phosphate buffer solution, this antigen being preferably selected in the group of the antigens containing at least the fragment aa174-187 of the human VRS type A or B G protein , such as the antigens described above, preferably G2Na or MEFG2Na antigen;
b) dissolving PLGA copolymer in an organic solvent;
c) adding the dissolved antigen of step a) to the dissolved PLGA of step b) to produce a first emulsion;
d) adding the obtained emulsion of step c) to an aqueous solution comprising at least a viscosizer agent, a surfactant and an osmotic agent to produce a second emulsion comprising microspheres;
e) hardening and harvesting the resulting microspheres obtained at the step d); and
f) drying the obtained microspheres.

According to a aspect of the invention, the step b) consists in dissolving the antigen in a phosphate buffer at neutral pH at a concentration comprised between 15 to 20 % w/v.

According to another aspect of the method according to the invention, the organic solvent used in the step b) consists in a halogenated hydrocarbon, preferably in methylene chloride, the concentration of said methylene chloride being preferably comprised between 10 to 20 % w/v (weight/volume).

According to another aspect, the emulsion of the step c) is obtained by sonication and/or homogenization.

Still according to another preferred aspect of the method according to the invention, the viscosizer used in the step d) is polyvinyl pyrrolidone K90, with a concentration of said polyvinyl pyrrolidone K90 being selected between 1 and 25 % w/v.

In a preferred embodiment, the surfactant used in the step d) is a polysorbate, preferentially the polysorbate 80, wherein the concentration of said polysorbate 80 is selected between 0.1 and 0.5 % w/v.

In another preferred embodiment of the invention, the osmotic agent used in the step d) is selected between sodium chloride and mannitol. The preferred concentration of sodium chloride or mannitol is selected between 0.1 to 10 % w/v.

An uniqueness of the invention is that without using any blend of polymers or any combination of microparticles, the immunological response shows a two phase profile, which mimics a multiple dose schedule vaccination. *In vitro* release studies (see example 4), were found to show the following release profile: firstly, a sustained release phase for 14 days (from about 50 to 60 % antigen released), which is followed by no less than 14 days of absence of release. On the other hand, it is well known that an ultimate burst caused by a complete erosion of the microsphere matrix facilitates the entire release of the antigen from the matrix (confirmed by the second phase of immunological response). The biphasic immune response induced in rodents may be consequence of said release profile (a sustained release stage followed by a latent period), and said final burst. Initial diffusion through a pre-existing pore network in the microspheres and subsequent enhanced diffusion via erosion-induced pore enlargement and evolution may be the predominant antigen release mechanism.

Examples demonstrate the microparticles releasing MEFG2Na over a prolonged period and eliciting a biphasic immune response against RSV (up to 148 days).

Another aspect of the invention concerns the use of a composition according to the invention or microencapsulated antigen obtained by a method according to the invention, for the preparation of a vaccine or an immunogenic composition intended to prevent or to treat RSV infection.

More particularly, the microspheres contained in the composition allowed a controlled release of the antigen and/or a biphasic controlled release of the antigen.

According to the use of the invention for the preparation of a vaccine or an immunogenic composition, this composition comprises microencapsulated antigen in a single type of microspheres with a biphasic release of the antigen.

According to a particular preferred embodiment, the said antigen is selected in the group of the antigens containing at least the fragment aa174-187 of the human VRS type A or B G protein , such as the antigens described above, preferably G2Na or MEFG2Na antigen or any fragment thereof contained the fragment aa174-187.

More particularly, the invention claims the use of a vaccine composition or of a method for the preparation of a vaccine composition, as above described, for immunizing mammal against natural conformational antigen, particularly against RSV infections, or to produce antibodies directed against conformational antigen, such as RSV antigen.

The composition according to the invention can be administered by any way known by the man skilled in the art, with preferences for nasal, oral or parenteral administration routes.

In a preferred embodiment, the route of administration is the parenteral route.

The invention will be better understood in respect to the following examples and figures wherein:

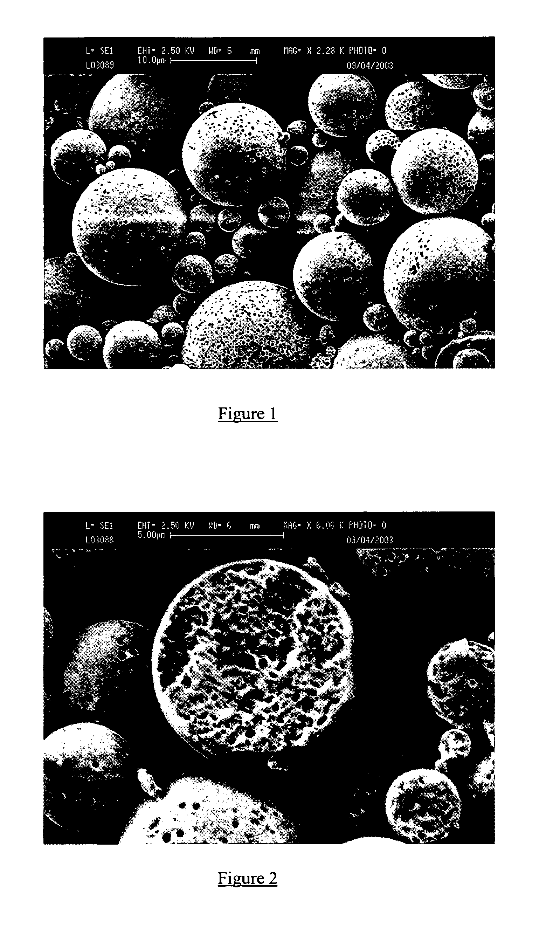

Figure 1 is a scanning electronic microscopy showing the outer morphology of antigen-loaded microspheres;
Figure 2 is a scanning electronic microscopy showing the inner morphology of antigen-loaded microspheres;

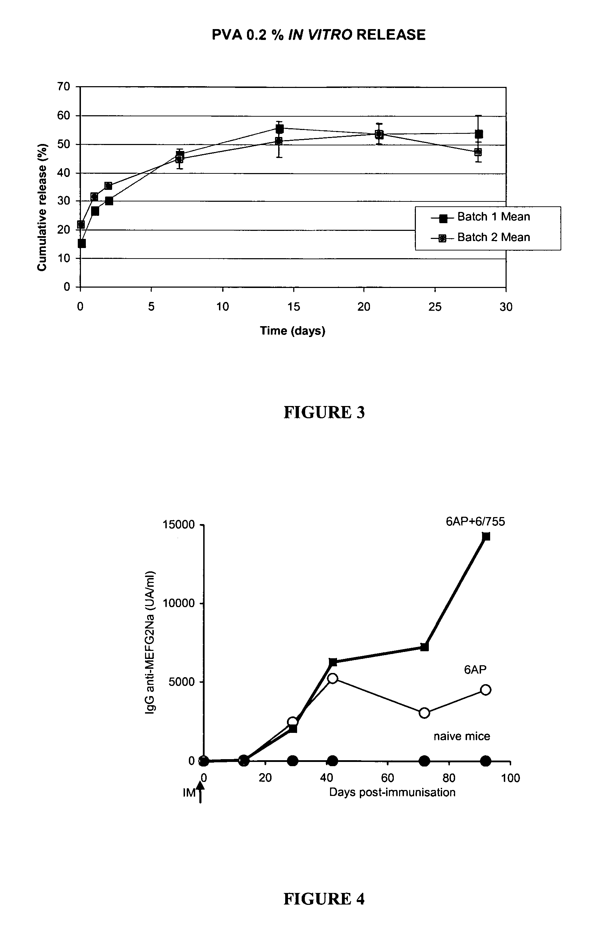

Figure 3 represents the cumulative antigen release in a solution of 0.2% (w/v) polyvinyl alcohol for 28 days. Total protein determined by Bicinchoninic Acid Assay;
Figure 4 represents the increased immunogenicity conferred by the association of MEFG2Na microencapsulated in PLGA microspheres to MEFG2Na adsorbed onto Adju-Phos in naive mice, wherein "6AP" means mice immunized with a single IM injection of 6µg MEFG2Na adsorbed onto Adju-Phos; and "6AP+6/755" means mice immunized with a single IM injection of 6µg MEFG2Na adsorbed onto Adju-Phos plus 6µg of MEFG2Na microencapsulated in PLGA microspheres (PLGA 75:25 of intrinsic viscosity of about 0.6dL/g);

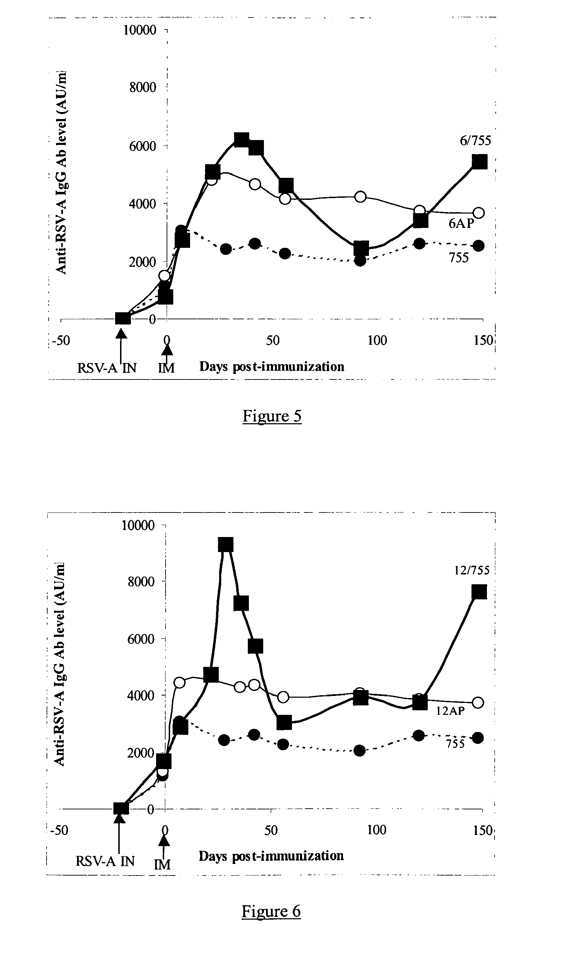

Figure 5 represents the biphasic antibody response conferred by the single injection of 6 µg MEFG2Na microencapsulated in PLGA microspheres in RSV-A-primed mice, wherein "755" means mice immunized with a single IM injection of blank (without MEFG2Na) PLGA microspheres (PLGA 75:25 of intrinsic viscosity of about 0.6 dl/g); "6AP" means mice immunized with a single IM injection of 6 µg MEFG2Na adsorbed onto Adju-Phos; and "6/755" means mice immunized with a single IM injection of 6µg MEFG2Na microencapsulated in PLGA microspheres (PLGA 75:25 of intrinsic viscosity of about 0.6 dl/g);
Figure 6 represents the biphasic antibody response conferred by the single injection of 12µg MEFG2Na microencapsulated in PLGA microspheres in RSV-A-primed mice, wherein "755" means mice immunized with a single IM injection of blank (without MEFG2Na) PLGA microspheres (PLGA 75:25 of intrinsic viscosity of about 0.6dL/g); "12AP" means mice immunized with a single IM injection of 12 µg MEFG2Na adsorbed onto Adju-Phos and; "12/755" means mice immunized with a single IM injection of 12µg MEFG2Na microencapsulated in PLGA microspheres (PLGA 75:25 of intrinsic viscosity of about 0.6dL/g);

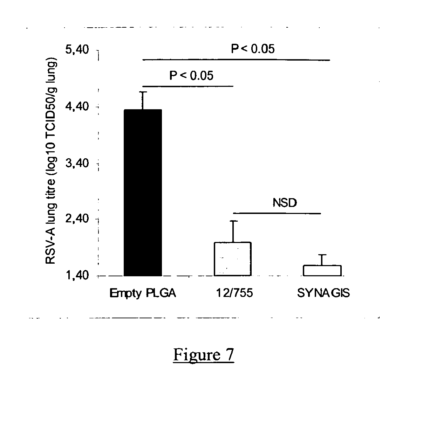

Figure 7 illustrates the protective efficacy obtained with the product of the invention, wherein « empty PLGA » means blank PLGA, i.e. PLGA microspheres without MEFG2Na; "12/755" means mice immunized with a single IM injection of 12 µg of MEFG2Na microencapsulated in PLGA microspheres (PLGA 75:25 of intrinsic viscosity of about 0.6dL/g); and "SYNAGIS" means cotton rats receiving 1.25mg/kg SYNAGIS (palivizumab: humanized monoclonal antibody anti-RSV fusion protein) 24h prior challenge; "P<0.05" means there is a statistical difference between groups compared; and "NSD" means not statistically different.

### Example 1: Production of recombinant protein MEFG2Na

Gene encoding of MEFG2Na (104 aa) was subcloned into a expression vector based on Trp promoter. It was transformed into ICONE 200 a *Escherichia coli* host, a derivative of RV 308 strain (Chevalet *et al.,* 2000, Biotechnol. Bioeng. **69**:351-358). The vector and the host bacteria were chosen in order to control tightly the expression of the recombinant protein in the late log phase of the growth.

Fermentation process: the recombinant strains were grown in a well defined synthetic medium using Glycerol as Carbone source. When OD (620nm) = 110, the expression was induced by addition of IAA (Indole acrylic acid). At the end of the culture, cells were subsequently sedimented (by centrifugation), washed and stored at -30 °C. The biomass obtained was approx. 60g of dry weight cells and the interested protein concentration was approx. 3 g of MEFG2Na/ liter of culture.

Downstream processing: cells were solubilised in a buffer containing Guanidine HCl. The solution was centrifuged and the supernatant was filtered (0.22 µm). Three steps purification was performed: cation exchange (Fractogel EMD SE Hicap), gel filtration (Superdex 75) and anion exchange (DEAE Sepharose). The purified proteins was filtered and stored in sterile bags at -30 °C.

### Example 2: Microncapsulating MEFG2Na in PLGA microspheres

As a general method, microencapsulation of MEFG2Na antigen is performed at room temperature and at atmospheric pressure according to the following protocol:
a) PLGA copolymer of the desired ratio lactide:glycolide (more preferably about 50:50 or 80:20 molar ratio) and inherent viscosity (generally in the range of 0.15 to 0.70 dl/g) is dissolved in an organic solvent (generally in a halogenated hydrocarbon, more preferably in methylene chloride) to the desired concentration (generally from about 10 to 20 % w/v). No other excipient, such as a release modulator, is added;
b) MEFG2Na is dissolved in a phosphate buffer at neutral pH at a desired concentration (generally from about 15 to 20 % w/v);
c) an excipient to stabilize the antigen may be added to an aliquot of the latter concentrated antigen solution (b). Examples of excipients used include sugars (such as mannitol or trehalose), amino acids (such as L-arginine), basic salts (such as magnesium hydroxide, calcium hydroxide, zinc carbonate or magnesium carbonate), surfactants (such as polysorbates) or polymers acting as stabilizers (such as polyvinyl alcohol);
d) the latter aliquot containing antigen and excipient (c) is added to the polymer solution (a) while sonicating or homogenizing for a desired time to form a w/o emulsion. Essentially, such emulsion is comprised of an aqueous inner layer containing from about 0.5 to 5 % (w/w) of MEFG2Na to be microencapsulated;
e) after homogenization or sonication, the w/o emulsion is added to a reactor tank or a bath with a helical mixer, which contains an aqueous solution of a viscosizer agent (preferably polyvinyl pyrrolidone K90) at a desired concentration (generally from about 1 to 25 % w/v) to provide a desired viscosity of the solution, a surfactant (generally a polysorbate, more preferably polysorbate 80) at a desired concentration (generally from about 0.1 to 0.5 w/v) and an osmotic agent (preferably sodium chloride or mannitol) at a desired concentration (generally from about 0.1 to 10 % w/v);
f) the reaction tank or the bath with mixer containing the latter aqueous solution (e) and the first emulsion w/o (d) is mixed at a settled speed for a desired time to generate a second w/o/w emulsion;
g) a volume of purified water is added to the latter second emulsion w/o/w, while mixing at a settled speed;
h) the organic solvent is removed by a general method, which allows a rapid evaporation of the solvent, such as a continuous ramp flow, stripping or bubbling for a desired time;
i) the hardened microspheres are sieved and harvested by filtering and washing with purified water several times, and then lyophilized.

The MEFG2Na-loaded microspheres described in this invention are made of poly(D,L-lactide-co-glycolide) (PLGA), wherein the ratio of lactide to glycolide is from about 50:50 to 80:20 molar ratio (lactide:glycolide); the inherent viscosity of PLGA copolymers is about 0.15 to 0.70 dl/g; the average diameter of the microspheres is in the range of 5 to 35 µm; and the antigen experimental loading is from about 0.5 to 5 % (w/w). Electronic microphotographs show particles with spherical shape and a porous surface (figure 1).

The viscosity of the aqueous solution of step e) (i.e. the external phase of the second emulsion w/o/w) allows the generation of small microspheres, with an average diameter preferably less than 25 µm. The volume of purified water of step g) allows a rapid hardening of the resulting microspheres due to PLGA copolymer insolubility in water. The solvent removal methods employed in h) increase the emulsion-air interface thus reducing the solvent evaporation time necessary to harden the microspheres. The latter time along with lyophilization time must be sufficient to provide adequate levels of residual organic solvent. In addition, a rapid solvent removal allows a homogeneous dispersion of the antigen throughout the microsphere matrix.

Under electronic microscopy (figure 2), inner reservoirs distributed consistently within the polymeric matrix are showed, where the antigen molecules are entrapped and protected against degradation, thus maintaining their biological activity. The unexpected immune response induced in rodents may be attributable to such homogeneous distribution of antigen molecules within reservoirs, along with the surface microsphere morphology. Further, the inner and outer microsphere morphology may be attributable to the technique employed, specifically, double emulsion followed by solvent extraction/evaporation, and the physicochemical properties of the antigen preferred, explicitly MEFG2Na.

### Example 3: Conformational conservation of the antigen MEFG2Na in the microspheres

Generation of the antibody 18D1 (Plotnicky et al., Journal of Virology, July 1999, p.5637-5645).

MAb 18D1 was produced by immunizing BALB/c mice intraperitoneally (i.p.) with 50 µg of KLH-G1ΔCa in complete Freund's adjuvant (KLH-G1ΔCa corresponds to a chimeric peptide resulting from the fusion between the Keyhole Limpet Hemocyanin peptide carrier and the G1AΔC RSV A G protein antigen fragment aa174-187 wherein the cysteine residue in position 186 has been replaced by a serine and which comprises the disulfide bridge Cys₁₇₆-Cys₁₈₂, see document WO 95/27787 example 7). A second immunization was performed with incomplete Freund's adjuvant. Four days after a further, intravenous inoculation of 10 µg of KLH-G1ΔCa, the mice were killed and spleen cells were fused with SP2/0-Ag14 myeloma cells by using polyethylene glycol. The resulting hybridoma was screened for antibody secretion by ELISA with bovine serum albumin-conjugated G1ΔCa, as the coating antigen. A panel of hybridomas was selected on the basis of their peptide reactivity patterns and cloned three times by serial limiting dilution. One clone was expanded and injected into pristane primed mice to induce the production of ascitic fluid. Ascite fluids were purified, isotyped, and used in subsequent studies. Mab 18D1 is reactive with MEFG2Na and RSV-A.

### Conformational conservation

The antibody 18D1 has the capability to recognize the native cysteine noose.

By quantitative analysis of MEFG2Na after mild dissolution of MEFG2Na-loaded PLGA microspheres with acetone followed by precipitation of the proteins, it was found that the majority of microencapsulated MEFGNa was recovered. By ELISA with specific Mab 18D1 it was found that about 88 % of protein was structurally well-conformed. Results are represented in the following table 2.

**Table 2**

| EXTRACTION METHODOLOGY | **% MEFG2Na microencapsulated:** | | | % RATIO (well-conformed MEFG2Na) / (Total MEFG2Na) |
|---|---|---|---|---|
| | | **Total protein by µBCA** | **ELISA** | |
| 1°) Dissolution µ capsules in COLD ACETONE / precipitation MEFG2Na | Sample 1 | 1.03% | 0.94 % | 91 % |
| 2°/ Dissolution MEFG2Na in PBS / tween 0.1 % | Sample 2 | 1.25 % | 1.05% | 84 % |
| | **Mean** | **1** | **1.00 %** | **88 %** |

Furthermore, it was confirmed *in vivo* that MEFG2Na-loaded PLGA microspheres induced protective antibodies response that neutralized the RSV in animal (see animal experiments).

### Example 4: In vitro release experiments of MEFG2Na from PLGA microspheres

### Preparation of MEFG2Na-loaded PLGA microspheres

Two microsphere formulations were prepared by w/o/w emulsion/solvent evaporation method using a 75:25 PLGA copolymer, intrinsic viscosity of about 0.6 dl/g, which contains alkyl ester end groups. 173.3 ml of methylene chloride were added to 25.5 g of PLGA per duplicate. For the first microsphere formulation, 23.0 ml of a concentrated solution of MEFG2Na (18 mg/ml) in a phosphate buffer solution (pH 7.5) were added to one of the PLGA organic solution. For the second microsphere formulation, 24.5 ml of another concentrated solution of MEFG2Na (16.5 mg/ml) in a phosphate buffer solution (pH 7.5) were added to the other PLGA organic solution. Both mixtures were emulsified by sonication. Both primary emulsions were added to two external phases, each consisting of an aqueous solution of 8 % (w/v) polyvinyl pyrrolidone K90, 0.37 % (v/v) polysorbate 80 and 1% (w/v) sodium chloride and mixed at high speed in order to emulsify. A volume of purified water was added to both second emulsions and the methylene chloride was allowed to evaporate by bubbling. For both formulations, the obtained microspheres were sieved through a 90 µm mesh, collected by filtration through a 5 µm filter and washed thrice, and then freeze dried. Average diameter of first microsphere formulation was 14.8 and average diameter of second one was 14 µm; the microencapsulation efficiency being 68 and 69 %, respectively.

### Characterization of in vitro MEFG2Na release from microspheres

MEFG2Na release from microspheres after 28 days of incubation in a release medium was analyzed by Bicinchoninic Acid Assay (i.e. total protein determination).

150 mg of 2 different formulations of microspheres were incubated in 7.5 ml of an aqueous solution of 0.2 % (w/v) polyvinyl alcohol for 28 days. Incubation took place in rotating vials at 37 °C. At predetermined time points, 1ml aliquots were withdrawn and volume replaced. Microspheres were separated by centrifugation and the protein content of the supernatant (i.e. release media) analyzed.

Results were found to follow the following pattern (figure 3): i) a sustained release stage (50-60 % antigen released) for 14 days, and ii) a latent state over 14 days as a minimum. The first stage (i) includes a rapid release stage (15-20 % antigen released) for 2 h, which may be identified as a low initial burst, since the existence of such initial bursts is a common feature for sustained release formulations.

### Example 5: Evaluation of the RSV antibody response conferred by MEFG2Na microencapsulated in PLGA microspheres associated to MEFG2Na adsorbed onto Adju-Phos in RSV-A seronegative mice

### Materials and Methods

Microspheres were prepared by w/o/w emulsion/solvent evaporation method using a 75:25 PLGA, intrinsic viscosity of about 0.6 dl/g, which contains alkyl ester end groups. 5 ml of methylene chloride were added to 1.0g of PLGA. 0.75 ml of a concentrated solution of MEFG2Na (16 mg/ml) in a phosphate buffer solution (pH 7.5) were added to the PLGA organic solution and emulsified by sonication. This primary emulsion was added to an external phase consisting of an aqueous solution of 7 % (w/v) polyvinyl pyrrolidone K90, 0.25 (v/v) polysorbate 80 and 5 % (w/v) mannitol and mixed at high speed in order to emulsify. A volume of purified water was added to this second emulsion and the methylene chloride was allowed to evaporate by continuous ramp flow. The obtained microspheres were sieved through a 90µm mesh, collected by filtration through a 5 µm filter and washed thrice, and then freeze dried. Average diameter of microspheres was 14.8 µm, while microencapsulation efficiency was 88 %.

### Immunization of mice

Female 8 week old BALB/c mice (n = 6 /group) were immunized with one unique dose of µg MEFG2Na adsorbed onto Adju-phos, alone or co-administered with 6 µg MEFG2Na microencapsulated. Intramuscular immunization was carried out with a single injection of 100 µl of the appropriate preparation on day 0. Blood was sampled from all mice at different times (see below), and the serum was used for analysis of specific IgG using an ELISA technique.

### Measurement of the antibody response

Antibody response to MEFG2Na protein antigen was determined using an ELISA technique. MEFG2Na (2 µg/ml) in 0.05M sodium carbonate-bicarbonate buffer, pH 9.6 were added to 96 well microplates and incubated overnight at 4 °C. The wells were washed three times with phosphate buffered saline (PBS) pH 7.4, and were incubated at 37 °C for 2h with 200 µl of PBS containing 5 % gelatin. After washing, serial twofold dilutions of serum from 1:100 were incubated in plates for 2 h at 37 °C. The amount of bound immunoglobulin G (IgG) was estimated by the addition of a specific anti-mouse IgG-peroxidase conjugated antibody. After 1 h incubation at 37 °C the enzyme substrate {3,3',5,5'Tetramethylbenzidine} was added. The reaction was stopped by the addition of H₂SO₄ 1N. Absorbance at 450nm was then measured using a microplate spectrophotometer reader. A mouse reference serum was added to each plate to quantify specific IgG levels. Results, represented in the figure 4, are expressed in Arbitrary Units (AU)/ml.

### Results

A single injection of 6µg MEFG2Na adsorbed onto Adju-Phos triggered a specific antibody response measurable three weeks after injection, which peaked on day 42 and then slowly declined. In contrast, when 6 µg of MEFG2Na microencapsulated in PLGA microspheres were co-administered with 6 µg MEFG2Na adsorbed onto Adju-Phos, the anti-MEFG2Na peak antibody response was slightly enhanced on day 42 and further continue to dramatically increase, due to microsphere hydrolysis and thus MEFG2Na release. At the end of the study, the anti-MEFG2Na IgG antibody response was 3-fold increased as compared to 6 µg MEFG2Na adsorbed onto Adju-Phos.

### Example 6: Evaluation of the RSV antibody response conferred by 6 µg MEFG2Na microencapsulated in PLGA microspheres in RSV-A seropositive mice

### Materials and Methods

MEFG2Na-loaded PLGA microspheres were prepared by w/o/w emulsion/solvent evaporation method using a 75:25 PLGA copolymer, intrinsic viscosity of about 0.6 dl/g, which contains alkyl ester end groups. 173.3 ml of methylene chloride were added to 25.5 g of PLGA. 23 ml of a concentrated solution of MEFG2Na (18mg/ml) in a phosphate buffer solution (pH 7.5) were added to the PLGA organic solution and emulsified by sonication. This primary emulsion was added using a pump to an external phase consisting of an aqueous solution of 8 % (w/v) polyvinyl pyrrolidone K90, 0.37 (v/v) polysorbate 80 and 1 % (w/v) sodium chloride and mixed at high speed in order to emulsify. A volume of purified water was added to this second emulsion and the methylene chloride was allowed to evaporate by bubbling. The obtained microspheres were sieved through a 90 µm mesh, collected by filtration and washed thrice, and then freeze dried. Average diameter of microspheres was 14.8 µm, while microencapsulation efficiency was 68 %.

Blank microspheres were prepared by o/w emulsion/solvent evaporation method using a 75:25 PLGA copolymer, intrinsic viscosity of about 0.6 dl/g, which contains alkyl ester end groups. 170.0 ml of methylene chloride were added to 25.5 g of PLGA. This primary emulsion was added using a pump to an external phase consisting of an aqueous solution of 8% (w/v) polyvinyl pyrrolidone K90, 0.37 (v/v) polysorbate 80 and 1% (w/v) sodium chloride and mixed at high speed in order to emulsify. A volume of purified water was added to this second emulsion and the methylene chloride was allowed to evaporate by bubbling. The obtained microspheres were sieved through a 90µm mesh, collected by filtration and washed thrice, and then freeze dried. Average diameter of microspheres was 25 µm, while yield was 94 %.

### Immunization of mice

Groups of 10 female 8 week old BALB/c mice were used. They were primed by the intranasal route (10⁵ TCID₅₀ RSV-A, Long Strain) 3 weeks before immunization. RSV-A seroconversion was verified before immunization. Immunization consisted of a single injection of MEFG2Na, either adsorbed onto Adju-phos or microencapsulated, equivalent to 6 µg of MEFG2Na protein. Intramuscular immunization was carried out with a single injection of 100 µl of the appropriate preparation on day 0. Blood was sampled from all mice at different times (see below), and the serum was used for analysis of specific IgG using an ELISA technique.

### Measurement of the antibody response

Antibody response to RSV-A was determined using an ELISA technique. Purified inactivated RSV-A (10 µg/ml) in 0.05M sodium carbonate-bicarbonate buffer, pH 9.6 were added to 96 well microplates and incubated overnight at 37 °C. The wells were washed three times with phosphate buffered saline (PBS) pH 7.4, and were incubated at 37 °C for 2 h with 200 µl of PBS containing 5 % gelatin. After washing, serial twofold dilutions of serum from 1:100 were incubated in plates for 2h at 37 °C. The amount of bound immunoglobulin G (IgG) was estimated by the addition of a specific anti-mouse IgG-peroxidase conjugated antibody. After 1h incubation at 37 °C the enzyme substrate {3,3',5,5'Tetramethylbenzidine} was added. The reaction was stopped by the addition of H₂SO₄ 1N. Absorbance at 450nm was then measured using a microplate spectrophotometer reader. A mouse reference serum was added to each plate to quantify specific IgG levels. Results are expressed in Arbitrary Units (AU)/ml.

### Results

Results are represented at the figure 5. "755" gives the baseline production of RSV IgG antibodies (Ab) following the intranasal priming. A single injection of 6 µg MEFG2Na adsorbed onto Adju-Phos boosted the anti-RSV IgG Ab response which peaked 3 weeks after the injection, and then slowly declined. In contrast, a single IM administration of 6 µg MEFG2Na microencapsulated in PLGA microspheres not only resulted in a boosted anti-RSV IgG Ab response which also peaked on week 3 post-immunization, but also in the reactivation of the anti-RSV IgG Ab response 3 months after the injection, upon hydrolysis of MEFG2Na microencapsulated in PLGA microspheres. The intensity of the anti-RSV IgG Ab response obtained on the last day of the study was comparable to that observed at the peak response.

### Example 7: Evaluation of the RSV antibody response conferred by 12 µg MEFG2Na microencapsulated in PLGA microspheres in RSV-A seropositive mice

### Formulation

The formulations are the same as the ones used in the preceding example 6.

### Immunization of mice

Groups of 10 female 8 week old BALB/c mice were used. They were primed by the intranasal route (10⁵ TCID₅₀ RSV-A, Long Strain) 3 weeks before immunization. RSV-A seroconversion was verified before immunization. Immunization consisted of a single injection of MEFG2Na, either adsorbed onto Adju-phos or microencapsulated, equivalent to 12 µg of MEFG2Na protein. Intramuscular immunization was carried out with a single injection of 100 µl of the appropriate preparation on day 0. Blood was sampled from all mice at different times (see below), and the serum was used for analysis of specific IgG using an ELISA technique.

### Measurement of the antibody response

Antibody response to RSV-A was determined using an ELISA technique. Purified inactivated RSV-A (10 µg/ml) in 0.05 M sodium carbonate-bicarbonate buffer, pH 9.6 were added to 96 well microplates and incubated overnight at 37 °C. The wells were washed three times with phosphate buffered saline (PBS) pH 7.4, and were incubated at 37 °C for 2h with 200 µl of PBS containing 5 % gelatin. After washing, serial twofold dilutions of serum from 1:100 were incubated in plates for 2h at 37 °C. The amount of bound immunoglobulin G (IgG) was estimated by the addition of a specific anti-mouse IgG-peroxidase conjugated antibody. After 1h incubation at 37°C the enzyme substrate {3,3',5,5'Tetramethylbenzidine} was added. The reaction was stopped by the addition of H₂SO₄ 1N. Absorbance at 450nm was then measured using a microplate spectrophotometer reader. A mouse reference serum was added to each plate to quantify specific IgG levels. Results, represented at the figure 6, are expressed in Arbitrary Units (AU)/ml.

### Results

Similar results were obtained using a higher dose (12 µg) of MEFG2Na antigen. A single injection of 12µg MEFG2Na adsorbed onto Adju-Phos boosted the anti-RSV IgG Ab response which peaked 3 weeks after the injection, and then slowly declined. The intensity of the anti-RSV IgG Ab response was similar to that induced by 6 µg MEFG2Na adsorbed onto Adju-Phos. In contrast, a single IM administration of 12 µg MEFG2Na microencapsulated in PLGA microspheres not only resulted in a boosted anti-RSV IgG Ab response which also peaked on week 3 post-immunization, but also in the reactivation of the anti-RSV IgG Ab response 3 months after the injection, upon hydrolysis of MEFG2Na microencapsulated in PLGA microspheres. Again, the intensity of the anti-RSV IgG Ab response obtained on the last day of the study was comparable to that observed at the peak response. However, in contrast to MEFG2Na adsorbed onto Adju-Phos, the intensity of the anti-RSV IgG Ab response was higher after immunization with 12 µg MEFG2Na microencapsulated in PLGA microspheres as compared to 6 µg MEFG2Na microencapsulated in PLGA microspheres, demonstrating that the administration of MEFG2Na microencapsulated in PLGA microspheres resulted in a dose-dependent anti-RSV IgG Ab response.

### Example 8: Evaluation of the protective efficacy conferred by MEFG2Na microencapsulated in PLGA microspheres

### Materials and Methods

Microspheres were prepared by w/o/w emulsion/solvent evaporation method using a 75:25 PLGA copolymer, intrinsic viscosity of about 0.6 dl/g, which contains alkyl ester end groups. 173.3 ml of methylene chloride were added to 25.5 g of PLGA. 24.5 ml of a concentrated solution of MEFG2Na (16.5 mg/ml) in a phosphate buffer solution (pH 7.5) were added to the PLGA organic solution and emulsified by sonication. This primary emulsion was added using a pump to an external phase consisting of an aqueous solution of 8 % (w/v) polyvinyl pyrrolidone K90, 0.37 (v/v) polysorbate 80 and 1 % (w/v) sodium chloride and mixed at high speed in order to emulsify. A volume of purified water was added to this second emulsion and the methylene chloride was allowed to evaporate by bubbling. The obtained microspheres were sieved through a 90 µm mesh, collected by filtration and washed thrice, and then freeze dried. Average diameter of microspheres was 14.0 µm, while microencapsulation efficiency was 69 %.

### Immunization of mice

Animals sensitized to RSV and developing a anti-RSV-A antibody response are further protected against challenge. Thus, the protection efficacy of MEFG2Na microencapsulated in PLGA microspheres was performed in naive animals.

RSV-seronegative cotton rats received two IM doses of 12 µg MEFG2Na microencapsulated or blank PLGA. Immunizations were carried out on days 0 and 28. On day 141, a group of naive cotton rats was injected IM with 1.25 mg/kg SYNAGIS. On day 142, all animals were challenged with 10⁵ tissue culture infectious doses 50 (TCID₅₀) of RSV-A Long strain by intranasal instillation. They were sacrificed 5 days post-challenge to determine RSV-A level in lung homogenates. For this purpose, animals were anaesthetized and exsanguinated by cardiac puncture. Lungs were removed and placed in 1ml of medium. Ten percent suspensions of lung tissues in medium were prepared in dounce homogenisers and centrifuged. Lung homogenate supernatants were snap frozen and stored in liquid nitrogen until assayed for virus. Virus titers in these samples were determined by titration assays. Cytopathic effects were monitored on days 5 and 7 and virus titers calculated according to Kärber's method.

### Results

Results are illustrated by the figure 7. Cotton rats immunized with empty PLGA were not protected, as demonstrated by the high RSV-A lung titer. Cotton rats receiving 1.25 mg/kg SYNAGIS 24h prior challenge were protected : their RSV-A lung titer was statistically reduced as compared to rats receiving blank PLGA, and 3 out 6 animals had their lung sterile. After immunization with 12 µg of MEFG2Na microencapsulated in PLGA microspheres, the RSV-A lung titer was dramatically reduced by more than 2 log10, and thus were considered as protected. Four out of 7 animal had their lung sterile, without detectable RSV-A. Lung titer was statistically different from rats receiving empty PLGA, but not from SYNAGIS-receiving rats. Thus, 12 µg of MEFG2Na microencapsulated in PLGA microspheres is as efficient as SYNAGIS is reducing RSV-A lung titers.

## Claims

1. Composition comprising at least one antigen microencapsulated in a single population of microspheres, **characterized in that** said at least one antigen is a respiratory syncytial virus (RSV) antigen comprising a native conformational epitope.

2. Composition according to claim 1, wherein said conformational epitope is a fragment of at least 10 amino acids length of the G protein of the human RSV type A or B.

3. Composition according to claim 1 or 2, wherein said at least one antigen comprises the fragment aa174-187 of the G protein of the human RSV type A or B.

4. Composition according to one of claims 1 to 3, wherein said native conformational epitope comprises at least the disulfide bridge between the residue Cys in position 176 and the residue Cys in position 182 of the G protein of the human RSV type A or B.

5. Composition according to one of claims 1 to 4, wherein said at least one antigen comprises or is the antigen selected from the group consisting of:
a) the sequence aa174-187, aa130-230, aa171-187, aa140-190, aa158-190, aa140-192, aa140-194, aa140-196, aa140-198, aa140-200, of the G protein of the human RSV type A or B;
b) the peptide resulting from the covalent coupling between the fragment aa140-190 and aa144-158, or resulting from the covalent coupling between the fragment aa140-190 and aa144-159, or resulting from the covalent coupling between the fragment aa140-192 and aa144-158 of the G protein of the human RSV type A or B; and
c) the sequences as defined in a) or b) wherein the cysteine residue in position 173 and/or 186 has been replaced by a serine residue.

6. Composition according to claim 5 wherein said at least one antigen comprises or consists in the peptide G2Na having the sequence SEQ ID No. 1, or any fragment thereof comprising at least the fragment aa174-187.

7. Composition according to one of claims 1 to 6, wherein said at least one antigen is covalently coupled to the triamino-acids peptide having the sequence MEF.

8. Composition according to claim 7, wherein said at least one antigen comprises or consists in the peptide MEFG2Na having the sequence SEQ ID No. 2.

9. Composition according to one of the claims 1 to 8, wherein said at least one antigen is dispersed in the microspheres in the absence of any adjuvant.

10. Composition according to one of the claims 1 to 9 comprising a second antigen, said second antigen being microencapsulated in different microspheres than the ones used for the first antigen.

11. Composition according to one of the claims 1 to 9 comprising a second antigen, said second antigen being free or adsorbed on aluminum salts and/or aluminum phosphate and/or blank poly(D,L-lactide-co-glycolide) copolymer (PLGA) microspheres and/or antigen-loaded microspheres in the said composition.

12. Composition according to claim 10 or 11, wherein said second antigen comprises or consists in the protein F, or an active fragment thereof, of human RSV type A (SEQ ID No. 3), of human RSV typeB (SEQ ID No. 4), or of human RSV type A and B.

13. Composition according to one of claims 1 to 12, wherein said microspheres are made of biodegradable poly(D,L-lactide-co-glycolide) copolymer (PLGA).

14. Composition according to claim 13, wherein the molar ratio of lactide to glycolide is selected from 50:50 to 80:20.

15. Composition according to one of the claims 1 to 14, wherein said microspheres are presenting an average diameter comprised between 5 and 35 µm.

16. Composition according to one of the claims 1 to 15, wherein the inherent viscosity of the PLGA microspheres is comprised between 0.15 and 0.70 dl/g.

17. Composition according to one of the claims 1 to 16, wherein the microspheres are incorporating from about 0.5 to 5 %, preferably from about 0.5 to 2 % of antigen, preferably G2Na and/or MEFG2Na.

18. Composition according to one of the claims 1 to 17 further comprising a pharmaceutically acceptable carrier or excipient, as a medicament.

19. Composition according to one of the claims 1 to 18, as a vaccine or an immunogenic composition.

20. Method for producing a composition according to one of claims 1 to 19, **characterized in that** it comprises the following steps:
a) dissolving the RSV antigen in a phosphate buffer solution;
b) dissolving PLGA copolymer in an organic solvent;
c) adding the dissolved antigen of step a) to the dissolved PLGA of step b) to produce a first emulsion;
d) adding the obtained emulsion of step c) to an aqueous solution comprising at least a viscosizer agent, a surfactant and an osmotic agent to produce a second emulsion comprising microspheres;
e) hardening and harvesting the resulting microspheres obtained at the step d); and
f) drying the obtained microspheres.

21. Method according to claim 20, wherein the step b) consists in dissolving the antigen in a phosphate buffer at neutral pH at a concentration comprised between 15 to 20 % w/v.

22. Method according to one of the claims 20 or 21, wherein the organic solvent used in the step b) consists in a halogenated hydrocarbon, preferably in methylene chloride.

23. Method according to claim 22, wherein the concentration of methylene chloride is comprised between 10 to 20 % w/v.

24. Method according to one of the claims 20 to 23, wherein the emulsion of the step c) is obtained by sonication and/or homogenization.

25. Method according to the claim 20, wherein the viscosizer used in the step d) is polyvinyl pyrrolidone K90.

26. Method according to the claim 25, wherein the concentration of polyvinyl pyrrolidone K90 is selected between 1 and 25 % w/v.

27. Method according to the claim 20, wherein the surfactant used in the step d) is a polysorbate, preferentially the polysorbate 80.

28. Method according to the claim 27, wherein the concentration of polysorbate 80 is selected between 0.1 and 0.5 % w/v.

29. Method according to the claim 20, wherein the osmotic agent used in the step d) is selected between sodium chloride and mannitol.

30. Method according to the claim 29, wherein the concentration of sodium chloride or mannitol is selected between 0.1 to 10 % w/v.

31. Use of a composition according to one of claims 1 to 19 or a composition obtained by a method according to one of claims 20 to 30, for the preparation of a vaccine or immunogenic composition intended to prevent or to treat RSV infection.

32. Use of a composition according to claim 30, wherein the microspheres contained in the composition allowed a controlled release of the antigen.

33. Use of a composition according to claim 31 or 32, wherein the microspheres contained in the composition allowed a biphasic controlled release of the antigen.


SEQUENCE LISTING

<110> Pierre Fabre Médicament

<120> Novel vaccine composition for the treatment of respiratory
      infectious diseases

<140> EP07290319.8
<141> 2007-03-14

<130> D25281

<160> 5

<170> PatentIn version 3.3


<210> 1
<211> 101
<212> PRT
<213> respiratory syncytial virus

<400> 1

Thr Val Lys Thr Lys Asn Thr Thr Thr Thr Gln Thr Gln Pro Ser Lys
1               5                   10                  15
Pro Thr Thr Lys Gln Arg Gln Asn Lys Pro Pro Asn Lys Pro Asn Asn
            20                  25                  30
Asp Phe His Phe Glu Val Phe Asn Phe Val Pro Cys Ser Ile Cys Ser
        35                  40                  45
Asn Asn Pro Thr Cys Trp Ala Ile Cys Lys Arg Ile Pro Asn Lys Lys
    50                  55                  60
Pro Gly Lys Lys Thr Thr Thr Lys Pro Thr Lys Lys Pro Thr Phe Lys
65                  70                  75                  80
Thr Thr Lys Lys Asp His Lys Pro Gln Thr Thr Lys Pro Lys Glu Val
                85                  90                  95
Pro Thr Thr Lys Pro
            100


<210> 2
<211> 104
<212> PRT
<213> Artificial sequence

<220>
<223> Protein derived from fragment aa 130-230 of the G protein
      of the human RSV type A


<400> 2

Met Glu Phe Thr Val Lys Thr Lys Asn Thr Thr Thr Thr Gln Thr Gln
1               5                   10                  15
Pro Ser Lys Pro Thr Thr Lys Gln Arg Gln Asn Lys Pro Pro Asn Lys
            20                  25                  30
Pro Asn Asn Asp Phe His Phe Glu Val Phe Asn Phe Val Pro Cys Ser
        35                  40                  45
Ile Cys Ser Asn Asn Pro Thr Cys Trp Ala Ile Cys Lys Arg Ile Pro
    50                  55                  60
Asn Lys Lys Pro Gly Lys Lys Thr Thr Thr Lys Pro Thr Lys Lys Pro

65                  70                  75                  80
Thr Phe Lys Thr Thr Lys Lys Asp His Lys Pro Gln Thr Thr Lys Pro
                85                  90                  95
Lys Glu Val Pro Thr Thr Lys Pro
            100

<210> 3
<211> 574
<212> PRT
<213> respiratory syncytial virus

<400> 3

Met Glu Leu Pro Ile Leu Lys Ala Asn Ala Ile Thr Thr Ile Leu Ala
1               5                   10                  15
Ala Val Thr Phe Cys Phe Ala Ser Ser Gln Asn Ile Thr Glu Glu Phe
            20                  25                  30
Tyr Gln Ser Thr Cys Ser Ala Val Ser Lys Gly Tyr Leu Ser Ala Leu
        35                  40                  45
Arg Thr Gly Trp Tyr Thr Ser Val Ile Thr Ile Glu Leu Ser Asn Ile
    50                  55                  60
Lys Glu Asn Lys Cys Asn Gly Thr Asp Ala Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Ser Thr Pro Ala Ala Asn Asn Arg Ala Arg Arg Glu Leu Pro
            100                 105                 110
Arg Phe Met Asn Tyr Thr Leu Asn Asn Thr Lys Lys Thr Asn Val Thr
        115                 120                 125
Leu Ser Lys Lys Arg Lys Arg Arg Phe Leu Gly Phe Leu Leu Gly Val
    130                 135                 140
Gly Ser Ala Ile Ala Ser Gly Thr Ala Val Ser Lys Val Leu His Leu
145                 150                 155                 160
Glu Gly Glu Val Asn Lys Ile Lys Ser Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asp Lys Gln Leu Leu Pro Ile Val Asp
        195                 200                 205
Lys Gln Ser Cys Arg Ile Ser Asn Ile Glu Thr Val Ile Glu Phe Gln
    210                 215                 220
Gln Lys Asn Asn Arg Leu Leu Glu Ile Thr Arg Glu Phe Ser Val Asn
225                 230                 235                 240
Ala Gly Val Thr Thr Pro Val Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Asn Asn Val Gln Ile Val Arg Gln Gln Ser Tyr Ser Ile
        275                 280                 285
Met Ser Ile Ile Lys Glu Glu Val Leu Ala Tyr Val Val Gln Leu Pro
    290                 295                 300
Leu Tyr Gly Val Ile Asp Thr Pro Cys Trp Lys Leu His Thr Ser Pro
305                 310                 315                 320
Leu Cys Thr Thr Asn Thr Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Glu Thr Cys Lys Val Gln Ser Asn Arg Val Phe Cys Asp
        355                 360                 365
Thr Met Asn Ser Leu Thr Leu Pro Ser Glu Val Asn Leu Cys Asn Val

370                 375                 380
Asp Ile Phe Asn Pro Lys Tyr Asp Cys Lys Ile Met Thr Ser Lys Thr
385                 390                 395                 400
Asp Val Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Ala Ser Asn Lys Gly Val Asp
        435                 440                 445
Thr Val Ser Val Gly Asn Thr Leu Tyr Tyr Val Asn Lys Gln Glu Gly
    450                 455                 460
Lys Ser Leu Tyr Val Lys Gly Glu Pro Ile Ile Asn Phe Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Lys Ser Asp Glu Leu
            500                 505                 510
Leu His His Val Asn Ala Gly Lys Ser Thr Thr Asn Ile Met Ile Thr
        515                 520                 525
Thr Ile Ile Ile Val Ile Ile Val Ile Leu Leu Ser Leu Ile Ala Val
    530                 535                 540
Gly Leu Leu Leu Tyr Cys Lys Ala Arg Ser Thr Pro Val Thr Leu Ser
545                 550                 555                 560
Lys Asp Gln Leu Ser Gly Ile Asn Asn Ile Ala Phe Ser Asn
                565                 570


<210> 4
<211> 574
<212> PRT
<213> respiratory syncytial virus

<400> 4

Met Glu Leu Leu Ile His Arg Ser Ser Ala Ile Phe Leu Thr Leu Ala
1               5                   10                  15
Val Asn Ala Leu Tyr Leu Thr Ser Ser Gln Asn Ile Thr Glu Glu Phe
            20                  25                  30
Tyr Gln Ser Thr Cys Ser Ala Val Ser Arg Gly Tyr Phe Ser Ala Leu
        35                  40                  45
Arg Thr Gly Trp Tyr Thr Ser Val Ile Thr Ile Glu Leu Ser Asn Ile
    50                  55                  60
Lys Glu Thr Lys Cys Asn Gly Thr Asp Thr Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Asn Thr Pro Ala Ala Asn Asn Arg Ala Arg Arg Glu Ala Pro
            100                 105                 110
Gln Tyr Met Asn Tyr Thr Ile Asn Thr Thr Lys Asn Leu Asn Val Ser
        115                 120                 125
Ile Ser Lys Lys Arg Lys Arg Arg Phe Leu Gly Phe Leu Leu Gly Val
    130                 135                 140
Gly Ser Ala Ile Ala Ser Gly Ile Ala Val Ser Lys Val Leu His Leu
145                 150                 155                 160
Glu Gly Glu Val Asn Lys Ile Lys Asn Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asn Asn Arg Leu Leu Pro Ile Val Asn
        195                 200                 205
Gln Gln Ser Cys Arg Ile Ser Asn Ile Glu Thr Val Ile Glu Phe Gln

210                 215                 220
Gln Met Asn Ser Arg Leu Leu Glu Ile Thr Arg Glu Phe Ser Val Asn
225                 230                 235                 240
Ala Gly Val Thr Thr Pro Leu Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Ser Asn Val Gln Ile Val Arg Gln Gln Ser Tyr Ser Ile
        275                 280                 285
Met Ser Ile Ile Lys Glu Glu Val Leu Ala Tyr Val Val Gln Leu Pro
    290                 295                 300
Ile Tyr Gly Val Ile Asp Thr Pro Cys Trp Lys Leu His Thr Ser Pro
305                 310                 315                 320
Leu Cys Thr Thr Asn Ile Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Asp Thr Cys Lys Val Gln Ser Asn Arg Val Phe Cys Asp
        355                 360                 365
Thr Met Asn Ser Leu Thr Leu Pro Ser Glu Val Ser Leu Cys Asn Thr
    370                 375                 380
Asp Ile Phe Asn Ser Lys Tyr Asp Cys Lys Ile Met Thr Ser Lys Thr
385                 390                 395                 400
Asp Ile Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Val Ser Asn Lys Gly Val Asp
        435                 440                 445
Thr Val Ser Val Gly Asn Thr Leu Tyr Tyr Val Asn Lys Leu Glu Gly
    450                 455                 460
Lys Asn Leu Tyr Val Lys Gly Glu Pro Ile Ile Asn Tyr Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Arg Ser Asp Glu Leu
            500                 505                 510
Leu His Asn Val Asn Thr Gly Lys Ser Thr Thr Asn Ile Met Ile Thr
        515                 520                 525
Thr Ile Ile Ile Val Ile Ile Val Val Leu Leu Ser Leu Ile Ala Ile
    530                 535                 540
Gly Leu Leu Leu Tyr Cys Lys Ala Lys Asn Thr Pro Val Thr Leu Ser
545                 550                 555                 560
Lys Asp Gln Leu Ser Gly Ile Asn Asn Ile Ala Phe Ser Lys
                565                 570

<210> 5
<211> 298
<212> PRT
<213> respiratory syncytial virus

<400> 5

Met Ser Lys Asn Lys Asp Gln Arg Thr Ala Lys Thr Leu Glu Lys Thr
1               5                   10                  15
Trp Asp Thr Leu Asn His Leu Leu Phe Ile Ser Ser Gly Leu Tyr Lys
            20                  25                  30
Leu Asn Leu Lys Ser Ile Ala Gln Ile Thr Leu Ser Ile Leu Ala Met
        35                  40                  45
Ile Ile Ser Thr Ser Leu Ile Ile Thr Ala Ile Ile Phe Ile Ala Ser

50                  55                  60
Ala Asn His Lys Val Thr Leu Thr Thr Ala Ile Ile Gln Asp Ala Thr
65                  70                  75                  80
Ser Gln Ile Lys Asn Thr Thr Pro Thr Tyr Leu Thr Gln Asp Pro Gln
                85                  90                  95
Leu Gly Ile Ser Phe Ser Asn Leu Ser Glu Ile Thr Ser Gln Thr Thr
            100                 105                 110
Thr Ile Leu Ala Ser Thr Thr Pro Gly Val Lys Ser Asn Leu Gln Pro
        115                 120                 125
Thr Thr Val Lys Thr Lys Asn Thr Thr Thr Thr Gln Thr Gln Pro Ser
    130                 135                 140
Lys Pro Thr Thr Lys Gln Arg Gln Asn Lys Pro Pro Asn Lys Pro Asn
145                 150                 155                 160
Asn Asp Phe His Phe Glu Val Phe Asn Phe Val Pro Cys Ser Ile Cys
                165                 170                 175
Ser Asn Asn Pro Thr Cys Trp Ala Ile Cys Lys Arg Ile Pro Asn Lys
            180                 185                 190
Lys Pro Gly Lys Lys Thr Thr Thr Lys Pro Thr Lys Lys Pro Thr Phe
        195                 200                 205
Lys Thr Thr Lys Lys Asp His Lys Pro Gln Thr Thr Lys Pro Lys Glu
    210                 215                 220
Val Pro Thr Thr Lys Pro Thr Glu Glu Pro Thr Ile Asn Thr Thr Lys
225                 230                 235                 240
Thr Asn Ile Ile Thr Thr Leu Leu Thr Asn Asn Thr Thr Gly Asn Pro
                245                 250                 255
Lys Leu Thr Ser Gln Met Glu Thr Phe His Ser Thr Ser Ser Glu Gly
            260                 265                 270
Asn Leu Ser Pro Ser Gln Val Ser Thr Thr Ser Glu His Pro Ser Gln
        275                 280                 285
Pro Ser Ser Pro Pro Asn Thr Thr Arg Gln
    290                 295

SEQUENCE LISTING

<110> Pierre Fabre Médicament

<120> Novel vaccine composition for the treatment of respiratory
      infectious diseases

<130> D25281

<160> 5

<170> PatentIn version 3.3

<210> 1
<211> 101
<212> PRT
<213> respiratory syncytial virus

<400> 1

Thr Val Lys Thr Lys Asn Thr Thr Thr Thr Gln Thr Gln Pro Ser Lys
1               5                   10                  15
Pro Thr Thr Lys Gln Arg Gln Asn Lys Pro Pro Asn Lys Pro Asn Asn
            20                  25                  30
Asp Phe His Phe Glu Val Phe Asn Phe Val Pro Cys Ser Ile Cys Ser
        35                  40                  45
Asn Asn Pro Thr Cys Trp Ala Ile Cys Lys Arg Ile Pro Asn Lys Lys
    50                  55                  60
Pro Gly Lys Lys Thr Thr Thr Lys Pro Thr Lys Lys Pro Thr Phe Lys
65                  70                  75                  80
Thr Thr Lys Lys Asp His Lys Pro Gln Thr Thr Lys Pro Lys Glu Val
                85                  90                  95
Pro Thr Thr Lys Pro
            100

<210> 2
<211> 104
<212> PRT
<213> Artificial sequence

<400> 2

Met Glu Phe Thr Val Lys Thr Lys Asn Thr Thr Thr Thr Gln Thr Gln
1               5                   10                  15
Pro Ser Lys Pro Thr Thr Lys Gln Arg Gln Asn Lys Pro Pro Asn Lys
            20                  25                  30
Pro Asn Asn Asp Phe His Phe Glu Val Phe Asn Phe Val Pro Cys Ser
        35                  40                  45
Ile Cys Ser Asn Asn Pro Thr Cys Trp Ala Ile Cys Lys Arg Ile Pro
    50                  55                  60
Asn Lys Lys Pro Gly Lys Lys Thr Thr Thr Lys Pro Thr Lys Lys Pro
65                  70                  75                  80
Thr Phe Lys Thr Thr Lys Lys Asp His Lys Pro Gln Thr Thr Lys Pro
                85                  90                  95
Lys Glu Val Pro Thr Thr Lys Pro
            100

<210> 3

<211> 574
<212> PRT
<213> respiratory syncytial virus

<400> 3

Met Glu Leu Pro Ile Leu Lys Ala Asn Ala Ile Thr Thr Ile Leu Ala
1               5                   10                  15
Ala Val Thr Phe Cys Phe Ala Ser Ser Gln Asn Ile Thr Glu Glu Phe
            20                  25                  30
Tyr Gln Ser Thr Cys Ser Ala Val Ser Lys Gly Tyr Leu Ser Ala Leu
        35                  40                  45
Arg Thr Gly Trp Tyr Thr Ser Val Ile Thr Ile Glu Leu Ser Asn Ile
    50                  55                  60
Lys Glu Asn Lys Cys Asn Gly Thr Asp Ala Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Ser Thr Pro Ala Ala Asn Asn Arg Ala Arg Arg Glu Leu Pro
            100                 105                 110
Arg Phe Met Asn Tyr Thr Leu Asn Asn Thr Lys Lys Thr Asn Val Thr
        115                 120                 125
Leu Ser Lys Lys Arg Lys Arg Arg Phe Leu Gly Phe Leu Leu Gly Val
    130                 135                 140
Gly Ser Ala Ile Ala Ser Gly Thr Ala Val Ser Lys Val Leu His Leu
145                 150                 155                 160
Glu Gly Glu Val Asn Lys Ile Lys Ser Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asp Lys Gln Leu Leu Pro Ile Val Asp
        195                 200                 205
Lys Gln Ser Cys Arg Ile Ser Asn Ile Glu Thr Val Ile Glu Phe Gln
    210                 215                 220
Gln Lys Asn Asn Arg Leu Leu Glu Ile Thr Arg Glu Phe Ser Val Asn
225                 230                 235                 240
Ala Gly Val Thr Thr Pro Val Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Asn Asn Val Gln Ile Val Arg Gln Gln Ser Tyr Ser Ile
        275                 280                 285
Met Ser Ile Ile Lys Glu Glu Val Leu Ala Tyr Val Val Gln Leu Pro
    290                 295                 300
Leu Tyr Gly Val Ile Asp Thr Pro Cys Trp Lys Leu His Thr Ser Pro
305                 310                 315                 320
Leu Cys Thr Thr Asn Thr Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Glu Thr Cys Lys Val Gln Ser Asn Arg Val Phe Cys Asp
        355                 360                 365
Thr Met Asn Ser Leu Thr Leu Pro Ser Glu Val Asn Leu Cys Asn Val
    370                 375                 380
Asp Ile Phe Asn Pro Lys Tyr Asp Cys Lys Ile Met Thr Ser Lys Thr
385                 390                 395                 400
Asp Val Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Ala Ser Asn Lys Gly Val Asp

435                 440                 445
Thr Val Ser Val Gly Asn Thr Leu Tyr Tyr Val Asn Lys Gln Glu Gly
    450                 455                 460
Lys Ser Leu Tyr Val Lys Gly Glu Pro Ile Ile Asn Phe Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Lys Ser Asp Glu Leu
            500                 505                 510
Leu His His Val Asn Ala Gly Lys Ser Thr Thr Asn Ile Met Ile Thr
        515                 520                 525
Thr Ile Ile Ile Val Ile Ile Val Ile Leu Leu Ser Leu Ile Ala Val
    530                 535                 540
Gly Leu Leu Leu Tyr Cys Lys Ala Arg Ser Thr Pro Val Thr Leu Ser
545                 550                 555                 560
Lys Asp Gln Leu Ser Gly Ile Asn Asn Ile Ala Phe Ser Asn
                565                 570

<210> 4
<211> 574
<212> PRT
<213> respiratory syncytial virus

<400> 4

Met Glu Leu Leu Ile His Arg Ser Ser Ala Ile Phe Leu Thr Leu Ala
1               5                   10                  15
Val Asn Ala Leu Tyr Leu Thr Ser Ser Gln Asn Ile Thr Glu Glu Phe
            20                  25                  30
Tyr Gln Ser Thr Cys Ser Ala Val Ser Arg Gly Tyr Phe Ser Ala Leu
        35                  40                  45
Arg Thr Gly Trp Tyr Thr Ser Val Ile Thr Ile Glu Leu Ser Asn Ile
    50                  55                  60
Lys Glu Thr Lys Cys Asn Gly Thr Asp Thr Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Asn Thr Pro Ala Ala Asn Asn Arg Ala Arg Arg Glu Ala Pro
            100                 105                 110
Gln Tyr Met Asn Tyr Thr Ile Asn Thr Thr Lys Asn Leu Asn Val Ser
        115                 120                 125
Ile Ser Lys Lys Arg Lys Arg Arg Phe Leu Gly Phe Leu Leu Gly Val
    130                 135                 140
Gly Ser Ala Ile Ala Ser Gly Ile Ala Val Ser Lys Val Leu His Leu
145                 150                 155                 160
Glu Gly Glu Val Asn Lys Ile Lys Asn Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asn Asn Arg Leu Leu Pro Ile Val Asn
        195                 200                 205
Gln Gln Ser Cys Arg Ile Ser Asn Ile Glu Thr Val Ile Glu Phe Gln
    210                 215                 220
Gln Met Asn Ser Arg Leu Leu Glu Ile Thr Arg Glu Phe Ser Val Asn
225                 230                 235                 240
Ala Gly Val Thr Thr Pro Leu Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Ser Asn Val Gln Ile Val Arg Gln Gln Ser Tyr Ser Ile

        275                 280                 285
Met Ser Ile Ile Lys Glu Glu Val Leu Ala Tyr Val Val Gln Leu Pro
    290                 295                 300
Ile Tyr Gly Val Ile Asp Thr Pro Cys Trp Lys Leu His Thr Ser Pro
305                 310                 315                 320
Leu Cys Thr Thr Asn Ile Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Asp Thr Cys Lys Val Gln Ser Asn Arg Val Phe Cys Asp
        355                 360                 365
Thr Met Asn Ser Leu Thr Leu Pro Ser Glu Val Ser Leu Cys Asn Thr
    370                 375                 380
Asp Ile Phe Asn Ser Lys Tyr Asp Cys Lys Ile Met Thr Ser Lys Thr
385                 390                 395                 400
Asp Ile Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Val Ser Asn Lys Gly Val Asp
        435                 440                 445
Thr Val Ser Val Gly Asn Thr Leu Tyr Tyr Val Asn Lys Leu Glu Gly
    450                 455                 460
Lys Asn Leu Tyr Val Lys Gly Glu Pro Ile Ile Asn Tyr Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Arg Ser Asp Glu Leu
            500                 505                 510
Leu His Asn Val Asn Thr Gly Lys Ser Thr Thr Asn Ile Met Ile Thr
        515                 520                 525
Thr Ile Ile Ile Val Ile Ile Val Val Leu Leu Ser Leu Ile Ala Ile
    530                 535                 540
Gly Leu Leu Leu Tyr Cys Lys Ala Lys Asn Thr Pro Val Thr Leu Ser
545                 550                 555                 560
Lys Asp Gln Leu Ser Gly Ile Asn Asn Ile Ala Phe Ser Lys
                565                 570

<210> 5
<211> 298
<212> PRT
<213> respiratory syncytial virus

<400> 5

Met Ser Lys Asn Lys Asp Gln Arg Thr Ala Lys Thr Leu Glu Lys Thr
1               5                   10                  15
Trp Asp Thr Leu Asn His Leu Leu Phe Ile Ser Ser Gly Leu Tyr Lys
            20                  25                  30
Leu Asn Leu Lys Ser Ile Ala Gln Ile Thr Leu Ser Ile Leu Ala Met
        35                  40                  45
Ile Ile Ser Thr Ser Leu Ile Ile Thr Ala Ile Ile Phe Ile Ala Ser
    50                  55                  60
Ala Asn His Lys Val Thr Leu Thr Thr Ala Ile Ile Gln Asp Ala Thr
65                  70                  75                  80
Ser Gln Ile Lys Asn Thr Thr Pro Thr Tyr Leu Thr Gln Asp Pro Gln
                85                  90                  95
Leu Gly Ile Ser Phe Ser Asn Leu Ser Glu Ile Thr Ser Gln Thr Thr
            100                 105                 110
Thr Ile Leu Ala Ser Thr Thr Pro Gly Val Lys Ser Asn Leu Gln Pro

        115                 120                 125
Thr Thr Val Lys Thr Lys Asn Thr Thr Thr Thr Gln Thr Gln Pro Ser
    130                 135                 140
Lys Pro Thr Thr Lys Gln Arg Gln Asn Lys Pro Pro Asn Lys Pro Asn
145                 150                 155                 160
Asn Asp Phe His Phe Glu Val Phe Asn Phe Val Pro Cys Ser Ile Cys
                165                 170                 175
Ser Asn Asn Pro Thr Cys Trp Ala Ile Cys Lys Arg Ile Pro Asn Lys
            180                 185                 190
Lys Pro Gly Lys Lys Thr Thr Thr Lys Pro Thr Lys Lys Pro Thr Phe
        195                 200                 205
Lys Thr Thr Lys Lys Asp His Lys Pro Gln Thr Thr Lys Pro Lys Glu
    210                 215                 220
Val Pro Thr Thr Lys Pro Thr Glu Glu Pro Thr Ile Asn Thr Thr Lys
225                 230                 235                 240
Thr Asn Ile Ile Thr Thr Leu Leu Thr Asn Asn Thr Thr Gly Asn Pro
                245                 250                 255
Lys Leu Thr Ser Gln Met Glu Thr Phe His Ser Thr Ser Ser Glu Gly
            260                 265                 270
Asn Leu Ser Pro Ser Gln Val Ser Thr Thr Ser Glu His Pro Ser Gln
        275                 280                 285
Pro Ser Ser Pro Pro Asn Thr Thr Arg Gln
    290                 295